# EUROPEAN PATENT APPLICATION

(11) **EP 2 870 909 A1**
(43) Date of publication of application: **13.05.2015**
(21) Application number: 14804821.8
(22) Date of filing: 20.03.2014
(51) Int. Cl.: A61B 1/00, A61B 1/04, G02B 23/24

(54) **ENDOSCOPE**

(30) Priority: 27.05.2013 JP 2013110928
(71) Applicant: Olympus Medical Systems Corp., Tokyo 151-0072 (JP)
(72) Inventor: SHIMADA Naoya, Tokyo 151-0072 (JP)
(74) Representative: Schicker, Silvia
(86) International application number: PCT/JP2014/057854
(87) International publication number: WO 2014/192386

(57) **Abstract**

An endoscope 1 includes: an insertion portion 2 in which a distal end portion 6 and a bending portion 7 are arranged; and an inclined face 24 that is formed in the distal end portion 6, the inclined face 24 being formed so as to inclinedly intersect with each side face adjoining a distal end face 20a of the distal end portion 6, and being formed substantially orthogonal to a ridge line 24a formed intersecting with the distal end face 20a and to a direction in which the bending portion 7 bends. By contacting against an inner wall of a curved portion of a duct 100 into which the insertion portion 2 is inserted, the inclined face varies a posture thereof so that the insertion portion 2 is rotated and a bending direction of the bending portion 7 matches a curving direction of the curved portion. Thus, the ability of the bending portion of the insertion portion to pass through a curved duct can be improved by means of a simple configuration that does not increase the manufacturing cost.

## Description

### Technical Field

The present invention relates to an endoscope including an insertion portion that includes a bending portion and that is inserted into a duct that curves.

### Background Art

As is known, endoscopes are widely used for performing observation and treatment and the like inside the body (inside a body cavity) of a living organism, or for performing inspection and repairs in plant facilities for industrial use. Such endoscopes have an insertion portion for inserting into a duct that curves.

A bending portion for improving the insertability into a duct is provided in the insertion portion of an endoscope. Such bending portions include a type that bends only in the two directions of upward and downward with respect to a photographed image that the endoscope photographs, and a type that bends in the four directions of upward, downward, left and right with respect to the aforementioned photographed image.

For example, Japanese Patent Application Laid-Open Publication No. 2004-351138 discloses an endoscope in which a bending portion is provided in an insertion portion. In the conventional endoscope disclosed therein, a spatulate projection portion is formed at a distal end of a distal end portion to enable smooth insertion of the insertion portion into a closed lumen such as the urethra.

In the spatulate projection portion provided in the aforementioned conventional endoscope, an inclined portion is formed by a surface that extends from the distal end of the distal end portion to the base. The inclined portion is located in a direction in which the bending portion bends, and pushes against an inner wall of the lumen when the bending portion bends.

Further, for example, in Japanese Patent Application Laid-Open Publication No. 2012-70934, technology is disclosed in which, to enable smooth insertion of the distal end of an endoscope insertion portion into a bile duct or a pancreatic duct which have a small diameter, an inclined face is provided at a distal end face of a distal end portion, and is formed in a cannonball shape.

In this connection, the insertion portion of an endoscope has a configuration such that the bending portion can only bend in specific directions as described above. Consequently, there is the problem that, depending on the rotational posture around the longitudinal axis at the time of insertion, it may not be possible for the bending portion of the insertion portion to pass through a duct that curves to a small degree, or because passage through a curved part is difficult, a large amount of force is required to push in the insertion portion.

In this regard, according to the endoscope technology disclosed in Japanese Patent Application Laid-Open Publication No. 2004-351138 and Japanese Patent Application Laid-Open Publication No. 2012-70934, although the distal end portion is formed in a spatula shape or a cannonball shape and the insertability of the distal end portion of the insertion portion into a narrow lumen or a constricted section or the like is improved, there is the problem that the respective technologies described above are not technologies that improve the passage through a curved duct of a bending portion of an insertion portion that only bends in specific directions, and furthermore the shapes and configurations of the above described distal end portions are complicated and the manufacturing cost is high.

The present invention has been made in consideration of the above described problems, and an object of the present invention is to provide an endoscope that, by means of a simple configuration that does not increase the manufacturing cost, can improve the ability of a bending portion of an insertion portion to pass through a curved duct.

### Disclosure of Invention

### Means for Solving the Problem

An endoscope according to one aspect of the present invention includes: an insertion portion in which a distal end portion and a bending portion that is bendable in a desired direction are arranged; and an inclined face that is formed in the distal end portion, the inclined face being formed so as to inclinedly intersect with each side face adjoining a distal end face of the distal end portion, and being formed substantially orthogonal to a ridge line formed intersecting with the distal end face and to a direction in which the bending portion bends, wherein, by contacting against an inner wall of a curved portion of a duct into which the insertion portion is inserted, the inclined face varies a posture thereof so that the insertion portion is rotated and a bending direction of the bending portion matches a curving direction of the curved portion.

According to the present invention described above, an endoscope can be provided that, by means of a simple configuration that does not increase the manufacturing cost, can improve the ability of a bending portion of an insertion portion to pass through a curved duct.

### Brief Description of the Drawings

Fig. 1 is a perspective view illustrating a configuration of an endoscope according to one embodiment of the present invention;
Fig. 2 is a front view illustrating a configuration of a distal end portion of an insertion portion;
Fig. 3 is a cross-sectional view illustrating an internal configuration of the distal end portion of the insertion portion;
Fig. 4 is a side view illustrating maximum bending states in the upward and downward directions of a bending portion of the insertion portion;
Fig. 5 is a cross-sectional view illustrating a state in which the distal end portion of the insertion portion has arrived at a curved portion of a duct;
Fig. 6 is a cross-sectional view along a line VI-VI in Fig. 5;
Fig. 7 is a cross-sectional view illustrating a state in which an inclined portion contacts against an inner wall of the curved portion of the duct, and the distal end portion rotates;
Fig. 8 is a cross-sectional view along a line VIII-VIII in Fig. 7;
Fig. 9 is a cross-sectional view illustrating a state in which the distal end portion of the insertion portion passes through the curved portion of the duct;
Fig. 10 is a cross-sectional view illustrating a state in which the distal end portion of the insertion portion has passed through the curved portion of the duct;
Fig. 11 is a side view illustrating an angle of the inclined portion formed in the distal end portion; and
Fig. 12 is a cross-sectional view illustrating the configuration of a distal end portion of a parent endoscope.

### Best Mode for Carrying Out the Invention

An embodiment of the present invention is described hereunder with reference to the drawings.

It should be noted that, in the following description, drawings that are based on the embodiment are schematic ones in which the relationship between the thickness and width of each portion, the thickness ratios between the respective portions and the like are different from those of actual portions, and the drawings may include portions in which the dimensional relationships and ratios are different from one another.

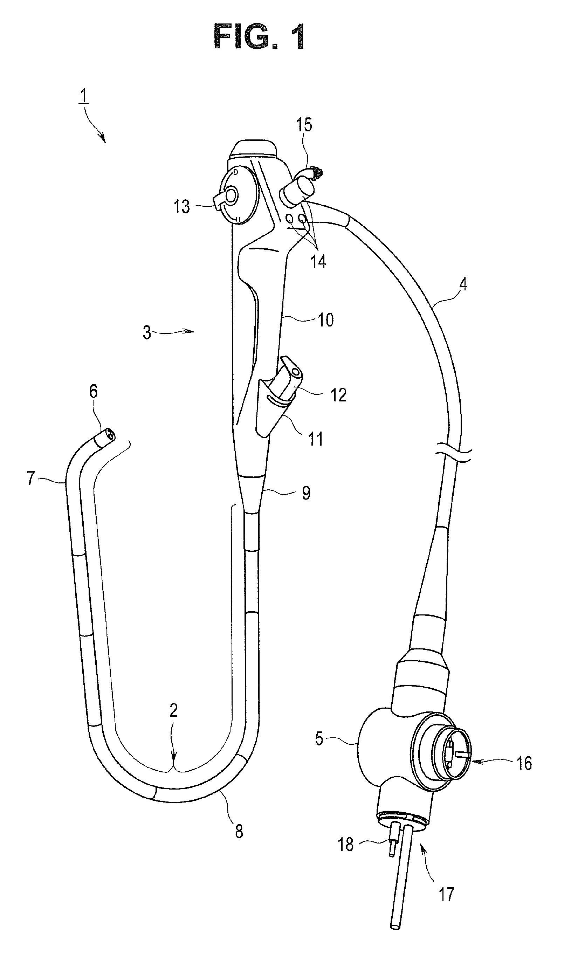

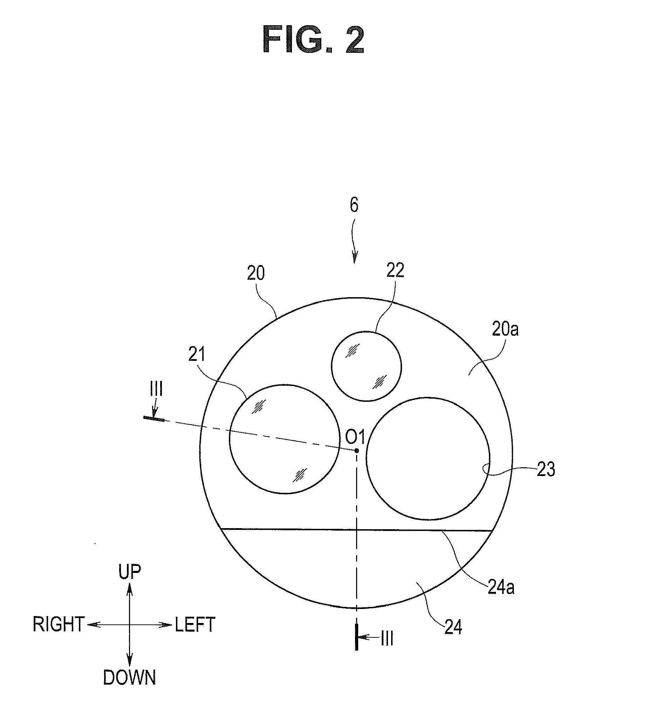

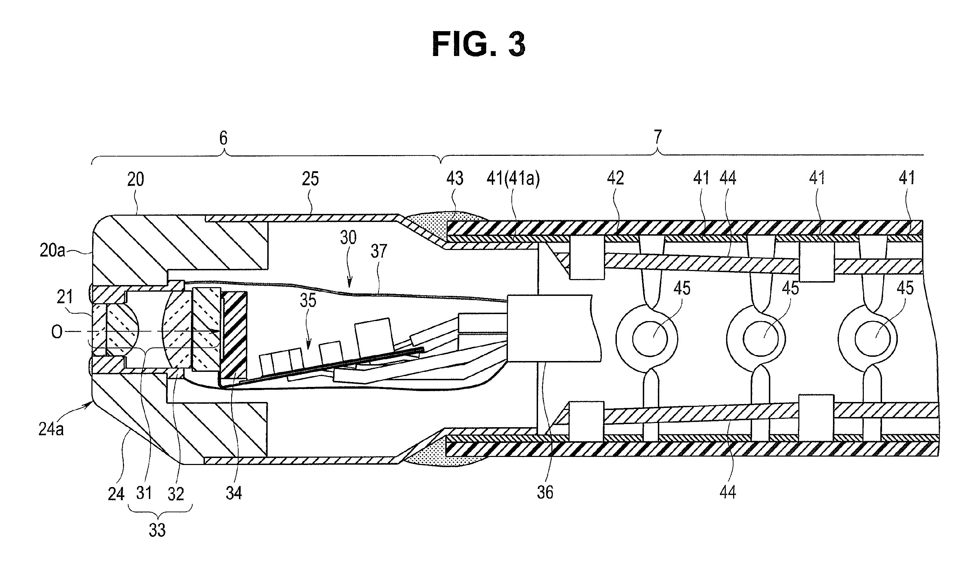

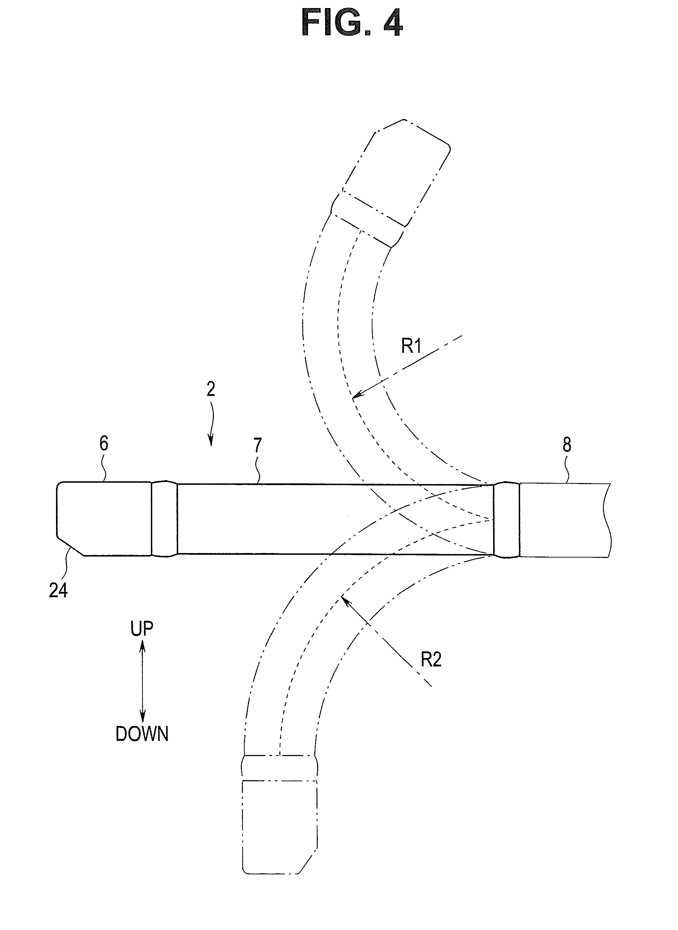

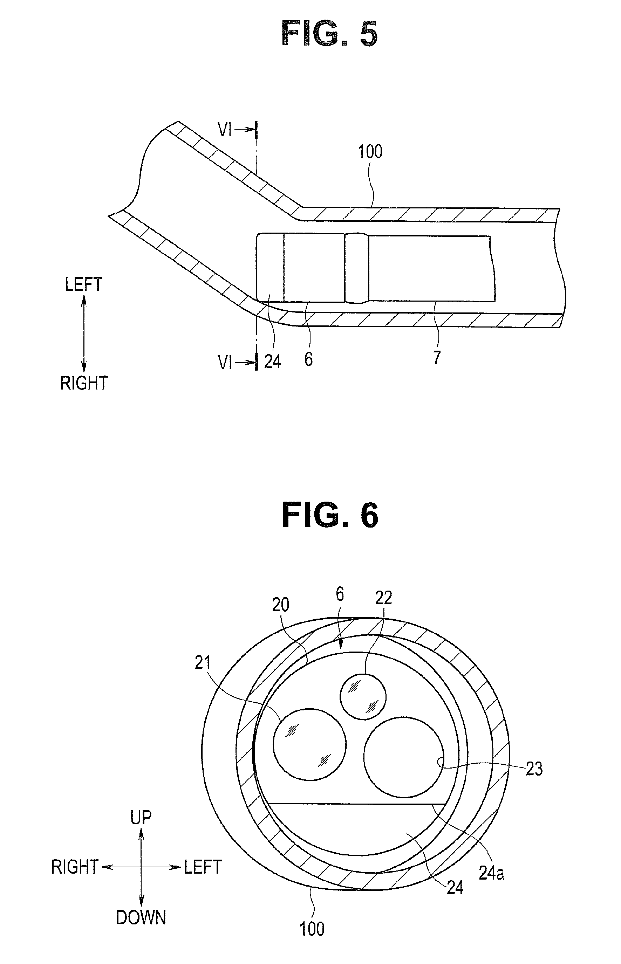

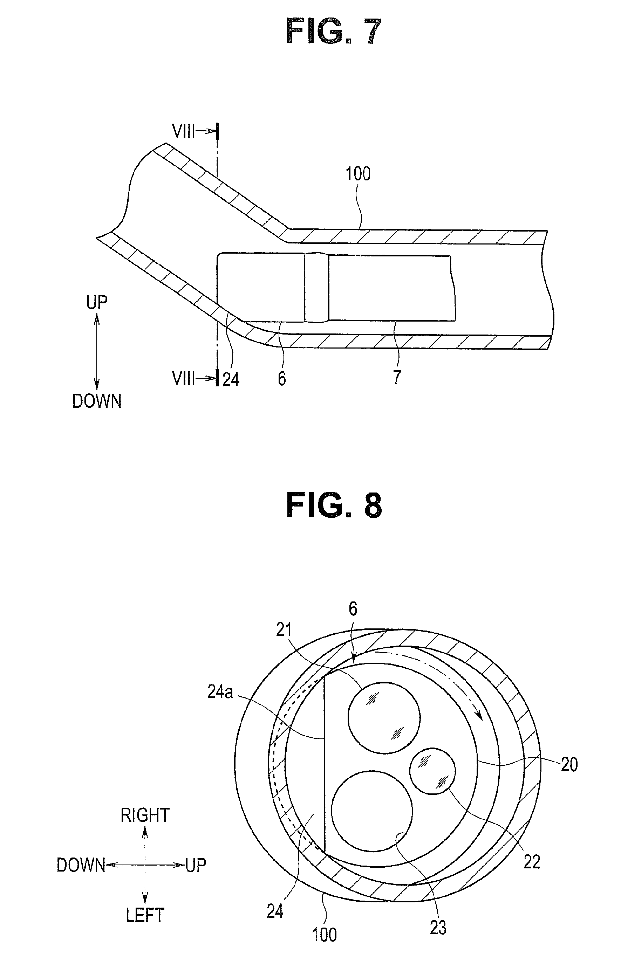

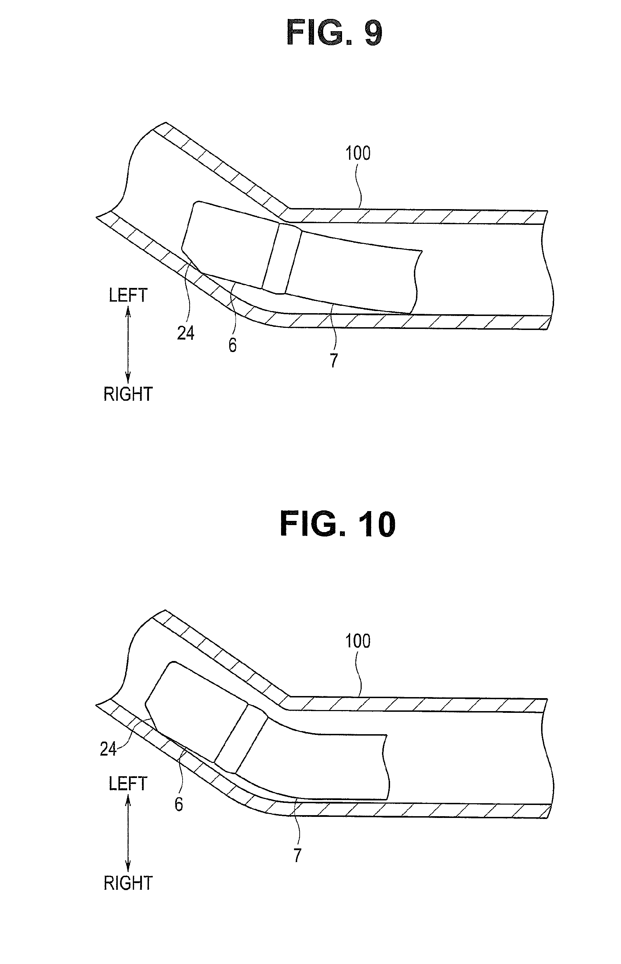

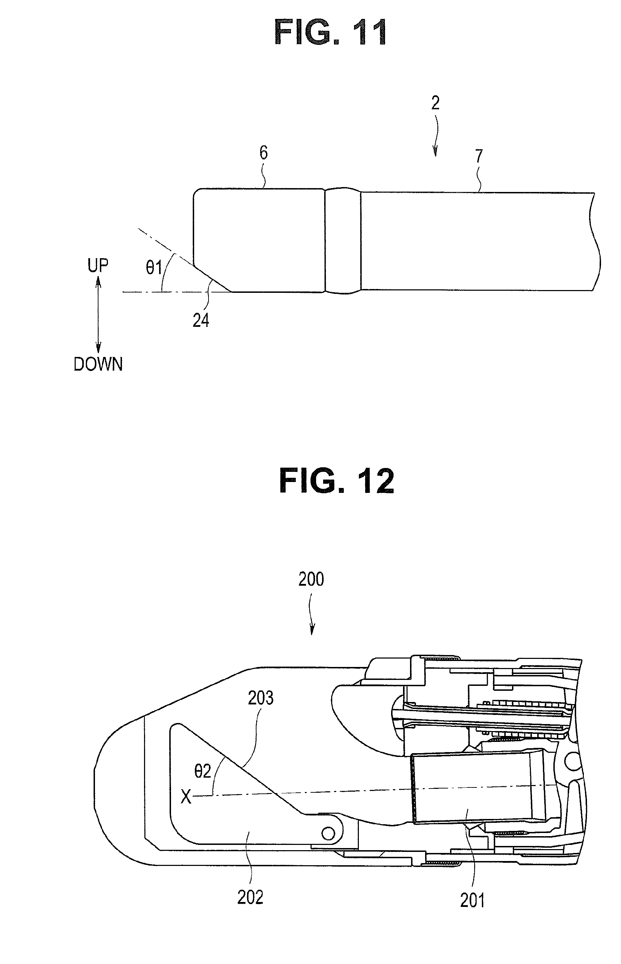

The drawings relate to one aspect to the present invention. Fig. 1 is a perspective view illustrating a configuration of an electronic endoscope. Fig. 2 is a front view illustrating a configuration of a distal end portion of an insertion portion. Fig. 3 is a cross-sectional view illustrating an internal configuration of the distal end portion of the insertion portion. Fig. 4 is a side view illustrating maximum bending states in the upward and downward directions of a bending portion of the insertion portion. Fig. 5 is a cross-sectional view illustrating a state in which the distal end portion of the insertion portion has arrived at a curved portion of a duct. Fig. 6 is a cross-sectional view along a line VI-VI in Fig. 5. Fig. 7 is a cross-sectional view illustrating a state in which an inclined portion contacts against an inner wall of the curved portion of the duct, and the distal end portion rotates. Fig. 8 is a cross-sectional view along a line VIII-VIII in Fig. 7. Fig. 9 is a cross-sectional view illustrating a state in which the distal end portion of the insertion portion passes through the curved portion of the duct. Fig. 10 is a cross-sectional view illustrating a state in which the distal end portion of the insertion portion has passed through the curved portion of the duct. Fig. 11 is a side view illustrating an angle of the inclined portion formed in the distal end portion. Fig. 12 is a cross-sectional view illustrating the configuration of a distal end portion of a parent endoscope.

As shown in Fig. 1, an endoscope 1 that is an electronic endoscope of the present embodiment is principally constituted by an insertion portion 2 formed in a shape of an elongated tube, an operation portion 3 connected to a proximal end of the insertion portion 2, a universal cord 4 that is an endoscope cable that extends from the operation portion 3, an endoscope connector 5 arranged at a distal end of the universal cord 4, and the like. Note that, in this case, although an electronic endoscope is mentioned as an example of the endoscope 1, the endoscope 1 is not limited thereto and may be a fiberscope that includes an ocular apparatus in the operation portion 3.

The insertion portion 2 is a flexible tubular member formed by connecting a distal end portion 6, a bending portion 7 and a flexible tube portion 8 in that order from the distal end side. Of these members, an image pickup unit that is an image pickup apparatus, described later, that contains image pickup means, and illumination means or the like are housed and disposed in the distal end portion 6.

The bending portion 7 is a mechanism region configured to be actively bendable in the two directions of up and down (UP-DOWN) as point-symmetric directions around a central axis O1 (see Fig. 2) of the insertion portion 2 through a rotational operation of a bending lever 13 among operation members of the operation portion 3.

Note that the bending portion 7 is not limited to a type that actively bends in the two directions of up and down, and may also be of a type that is bendable in four directions that include the left/right directions in addition to the up/down directions (capable of bending in all circumferential directions, UP-DOWN/LEFT-RIGHT, around the axis through vertical and horizontal operations), or may also be of a type that is bendable in only the upward (UP) direction. Furthermore, the bending portion 7 may be of a type that merely bends passively and does not have a mechanism whereby the bending portion 7 is actively bent by the bending lever 13.

The flexible tube portion 8 is a tubular member formed with flexibility so as to be passively flexible. In addition to a treatment instrument insertion channel that is described later, a signal cable bundle that is described later that extends from the image pickup apparatus contained in the distal end portion 6 and runs from the operation portion 3 to the inside of the universal cord 4, and a light guide bundle that is described later that guides an illuminating light from an light source apparatus and causes the illuminating light to exit from the distal end portion 6 and the like are inserted through the inside of the flexible tube portion 8 (in this case, these members are not shown in the drawings).

The operation portion 3 is constituted by: a bend preventing portion 9 that is provided on the distal end side to cover a proximal end of the flexible tube portion 8 and is connected to the flexible tube portion 8; a grasping portion 10 that is connected to the bend preventing portion 9 and which a user manually grasps when using the endoscope 1; operation means for operating various endoscope functions that are provided on an external surface of the grasping portion 10; a treatment instrument insertion portion 11; and a suction valve 15 or the like.

Examples of the operation means provided in the operation portion 3 include the bending lever 13 for performing bending operations of the bending portion 7, and a plurality of operation members 14 for performing air/water feeding operations or suction operations or operations corresponding to the image pickup means, the illumination means or the like.

The treatment instrument insertion portion 11 includes a treatment instrument insertion port for inserting various kinds of treatment instruments (not shown) and is a component that communicates with a treatment instrument insertion channel (not shown) through a branching member inside the operation portion 3.

A forceps plug 12 that is a cap member for opening/closing the treatment instrument insertion port is arranged in the treatment instrument insertion portion 11. The forceps plug 12 is configured to be detachable (replaceable) from the treatment instrument insertion portion 11. Note that, the treatment instrument insertion channel is configured to also communicate with the suction valve 15 by means of the branching member within the operation portion 3.

The universal cord 4 is a composite cable which extends through the inside of the insertion portion 2 from the distal end portion 6 of the insertion portion 2 to the operation portion 3, and through which a signal cable bundle and a light guide bundle that transmits an illuminating light from a light source apparatus (not shown) or the like are inserted.

The endoscope connector 5 includes, on a side face portion, an electrical connector portion 16 to which is connected a signal cable that connects the endoscope 1 with a video processor (not shown) that is an external device, and also includes a light source connector portion 17 to which are connected a light guide bundle, described later, and an electrical cable (not shown) that connect the endoscope 1 with a light source apparatus that is an external device, and an air/water feeding plug 18 that connects an air/water feeding tube (not shown) from an air/water feeding apparatus (not shown) that is an external device.

The configuration of the insertion portion 2 of the endoscope 1 of the present embodiment will now be described based on Fig. 2 and Fig. 3. Note that, in the following description, a description relating to known components of the insertion portion 2 is omitted. Note that, the upward, downward, left and right directions (UP/DOWN/LEFT/RIGHT) illustrated in Fig. 2 and Fig. 3 correspond to and match the upward, downward, left and right directions in an observation image that is photographed by an image pickup unit 30 that is described later.

As shown in Fig. 2, the distal end portion 6 of the insertion portion 2 includes a distal end rigid portion 20 that is a metal block body in which an observation window 21, an illuminating window 22 and a channel opening portion 23 are provided. In the distal end rigid portion 20, one section of an edge circumference portion on the distal end side is notched to form an inclined portion 24 as an inclined face. The configuration and action of the inclined portion 24 are described in detail later.

Further, as shown in Fig. 3, the distal end portion 6 has an exterior tube 25 that is fitted and fixed to the exterior of the proximal end portion of the distal end rigid portion 20. The image pickup unit 30 that is an image pickup apparatus and illumination means, not illustrated in the drawings, are provided in the distal end rigid portion 20. A treatment instrument channel, which is not illustrated in the drawings, is fitted and fixed inside the distal end rigid portion 20 so as to communicate with the channel opening portion 23.

Note that the treatment instrument channel passes through the inside of the insertion portion 2 from the distal end portion 6 of the insertion portion 2 to the operation portion 3, and communicates with the treatment instrument insertion port of the treatment instrument insertion portion 11 and the suction valve 15 illustrated in Fig. 1.

The image pickup unit 30 includes: a lens unit 33 in which a plurality of objective optical systems 31 including the observation window 21 are held in a lens holding barrel 32; a solid-state image pickup device 34 as image pickup means for detecting a subject image (observation image indicated by a photographing optical axis O in the drawing) that is formed by the objective optical systems 31; and a plurality of electronic components 35 mounted on an FPC that is electrically connected with the solid-state image pickup device 34 and the like.

A plurality of small signal cables extend from the electronic components 35, and a signal cable bundle 36 that is formed by bundling the small signal cables extends from the image pickup unit 30 to the proximal end side.

The signal cable bundle 36 that extends from the image pickup unit 30 is inserted through and disposed inside of the insertion portion 2, and is extended as far as the endoscope connector 5 via the operation portion 3 and the universal cord 4 shown in Fig. 1, and connected to the electrical connector portion 16 of the endoscope connector 5.

A heat-shrinkable tube 37 is provided from the proximal end portion of the lens unit 33 to the distal end portion of the signal cable bundle 36 so as to cover the plurality of electronic components 35 mounted on the FPC. An adhesive or the like for maintaining watertightness is filled inside the heat-shrinkable tube 37.

The configuration of the image pickup unit 30 of the present embodiment that is described above is similar to the known conventional configuration, and hence a detailed description of the remaining configuration thereof is omitted here.

The illumination means that is not illustrated in Fig. 3 has a configuration that includes a light guide bundle, not illustrated in the drawings, that is formed by bundling a plurality of fibers covered by an outer covering and that transmits an illuminating light, and which is fitted and retained in the distal end rigid portion 20 in a state in which a back face of the illuminating window 22 illustrated in Fig. 2 and a distal end face of the light guide bundle are opposingly disposed.

Note that the light guide bundle is inserted through and disposed inside the insertion portion 2, and is extended as far as the endoscope connector 5 via the operation portion 3 and the universal cord 4 shown in Fig. 1, and connected to the light source connector portion 17 of the endoscope connector 5.

In the bending portion 7 of the insertion portion 2, a plurality of bending pieces 41 that are made of metal are rotatably connected by pivoted portions 45 such as rivets, and a bending rubber 42 is covered over the outer circumference thereof to cover the plurality of bending pieces 41.

Note that the bending rubber 42 is preferably a multi-layered structure formed using a hard material on an outer layer side and a soft material on an inner layer side. As the bending rubber 42 having a multi-layered structure, for example, polyurethane or the like is used as the hard material on the outer layer side, and fluorocarbon rubber or the like is used as the soft material on the inner layer side.

By forming the bending rubber 42 as a multi-layered structure in this manner, and using a hard material on the outer layer side, the resistance characteristics of the surface are enhanced, and furthermore, by providing the bending rubber 42 with resilience by using a soft material on the inner layer side, the shock resistance of the bending portion 7 improves.

A bending piece disposed at the distalmost end among the plurality of bending pieces 41 described above is fitted and connected to the exterior of a small diameter portion at the proximal end of the exterior tube 25 connected to the proximal end portion of the distal end rigid portion 20 of the distal end portion 6. Note that, in Fig. 3, reference character "41 (41 a)" is assigned only to the bending piece disposed at the distalmost end among the plurality of bending pieces 41. In the following description, the relevant bending piece is referred to as "bending piece 41a".

A distal end portion of the bending rubber 42 is bonded by an adhesive portion 43 so as to maintain watertightness with an outer peripheral portion of the bending piece 41a. A proximal end portion of the bending rubber 42 is also bonded by an adhesive portion (not shown) so that a connecting portion between the bending piece 41 a and the flexible tube portion 8 is maintained in a watertight state.

In this case, the bending portion 7 is configured to be subjected to a bending operation in two directions, namely the upward/downward directions, of an observation image that the image pickup unit 30 picks up, in response to pulling/slackening of two bending operation wires 44 which are connected to the bending piece 41 a, through the plurality of bending pieces 41 in which the two bending operation wires 44 are inserted and held being rotated in a manner that utilizes the pivoted portions 45 as spindles.

The two bending operation wires 44 are inserted through the inside of an unshown coil sheath in a region extending from the proximal end of the bending portion 7 to the inside of the flexible tube portion 8 and the operation portion 3, and are connected to a sprocket (not shown) that operates in response to operation of the bending lever 13. Note that since a configuration that causes the bending portion 7 to bend is known, a detailed description of the remaining configuration thereof is omitted here.

Although not shown in the drawings, the flexible tube portion 8 of the insertion portion 2 is formed as, for example, a flexible tube body having a triple layer structure constituted by an outer-layer resin, an inner-layer resin, and metal braid of a metal reticular tube that is provided between the outer-layer resin and the inner-layer resin. Note that the metal braid may also be formed as a metal helical tube. Since the specific configuration of the flexible tube portion 8 is known, a detailed description of the remaining configuration thereof is omitted here.

In this connection, as shown in Fig. 4, with respect to the bending portion 7 of the present embodiment, a curvature radius R1 at which the bending portion 7 bends to the maximum in the upward (UP) direction is set to be less than a curvature radius R2 at which the bending portion 7 bends to the maximum in the downward (DOWN) direction. That is, the bending portion 7 is set so that the curvature when the bending portion 7 is bent to the maximum in the upward direction is less than when the bending portion 7 is bent to the maximum in the downward direction, and the bend is sharp.

Note that, as described above, in a case where the bending portion 7 is of a type that can bend in four directions (UP-DOWN/LEFT-RIGHT) that include the left/right directions in addition to the upward/downward directions also, a configuration is adopted so that the bending portion 7 can bend more in the upward direction than in the downward, left, and right directions. Further, in a case where bending portion 7 is of a type that can bend in only one direction which is the upward (UP) direction, the bending portion 7 does not bend in the downward, left, and right directions, and is in a rigid state with respect to those directions.

As shown in Fig. 2 and Fig. 3, the inclined portion 24 as an inclined face formed in the distal end portion 6 is formed so as to inclinedly intersect with a distal end face 20a of the distal end rigid portion 20 of the distal end portion 6 and respective side peripheral faces as side faces that serve as outer peripheral portions adjoining the distal end face 20a, and is substantially orthogonal to a ridge line 24a formed intersecting with the distal end face 20a and to the upward/downward (UP-DOWN) directions in which the bending portion 7 bends.

Further, the inclined portion 24 is formed to be parallel to the right/left (RIGHT-LEFT) directions in which the bending portion 7 bends, that is the side in the downward (DOWN) direction in which the bending portion 7 bends. That is, the inclined portion 24 is the downward direction of an observation image picked up by the image pickup unit 30 in the distal end rigid portion 20 of the distal end portion 6, and is formed parallel to the horizontal direction.

According to the endoscope 1 of the present embodiment configured as described above, for example, as shown in Fig. 5 and Fig. 6, when the distal end portion 6 of the insertion portion 2 that has been inserted into a duct 100 has come to a curved portion of the duct 100 which, in this case, curves to the left side, as the result of a manual operation performed by the user to push in the insertion portion 2, as shown in Fig. 7 and Fig. 8, the inclined portion 24 formed in the distal end portion 6 contacts against the inner wall of the curved portion of the duct 100 and the insertion portion 2 rotates along the inner wall of the duct 100.

That is, when the inclined portion 24 of the distal end portion 6 comes in contact with the inner wall of the curved portion of the duct 100, and the insertion portion 2 of the endoscope 1 is then pushed in further, a rotary force arises in the distal end portion 6 such that the inclined portion 24 is guided by the inner wall of the duct and rotates in accordance therewith.

Note that, in this case, the state is one in which the insertion portion 2 of the endoscope 1 is inserted into the duct 100 in a manner such that the front direction with respect to the page surface in Fig. 5 is the downward direction with respect to the direction in which the bending portion 7 bends.

Further, in the insertion portion 2, the upward, downward, left and right bending directions of the bending portion 7 rotate approximately 90° to enter the state illustrated in Fig. 8 from the state illustrated in Fig. 6. In this case, because the duct 100 curves to the left (LEFT) direction, the insertion portion 2 rotates counterclockwise as viewed from the user's side, and rotates clockwise as viewed facing the surface of the page of Fig. 6 and Fig. 8.

Thus, according to the endoscope 1 of the present embodiment, when the distal end portion 6 of the insertion portion 2 is passed through the curved portion of the duct 100, as a result of the inclined portion 24 formed in the distal end portion 6 contacting against the inner wall of the curved portion, the insertion portion 2 naturally rotates and the curving direction of the duct 100 and the upward (UP) direction in which the bending portion 7 bends match.

That is, since the inclined portion 24 of the distal end portion 6 is formed on the downward (DOWN) direction side in which the bending portion 7 bends, the insertion portion 2 is rotated so that the upward (UP) direction in which the bending portion 7 is bent with a small curvature radius matches the curving direction of the duct 100.

When the insertion portion 2 of the endoscope 1 is pushed in further in the direction of the deep part of the duct 100, as shown in Fig. 9, the posture of the distal end portion 6 inclines along the curved portion of the duct 100, and as shown in Fig. 10, the bending portion 7 bends passively in the upward direction and can easily pass through the curved portion of the duct 100.

Note that, the bending portion 7 of the insertion portion 2 has a configuration that only bends in specific directions, which in this case are the two directions of upward and downward (UP-DOWN), and is in a rigid state with respect to the left/right (LEFT-RIGHT) directions. Consequently, to facilitate passage of the bending portion 7 of the insertion portion 2 through the curved portion of the duct 100, it is sufficient to cause the upward/downward directions of the bending portion 7 to match the curving direction of the duct 100, and the bending portion 7 passively bends along the inner wall of the duct 100. By this means, using a small amount of force to push in the insertion portion 2, the user can easily cause the insertion portion 2 to pass through the curved portion of the duct 100.

As described above, according to the endoscope 1 of the present embodiment, the ability of the bending portion 7 of the insertion portion 2 to pass through the curved portion of the duct 100 is improved without increasing the manufacturing cost by a simple configuration in which the inclined portion 24 is formed on the lower side of the distal end portion 6.

Note that, with respect to the technology of the above described endoscope 1, although an example in which the insertion portion 2 is caused to pass through the curved portion of the duct 100 is described above, the present invention is not limited thereto. For example, the present invention is also applicable to a ureteroscope that is inserted via a tube body that curves, or to a child endoscope that is inserted into a channel of a parent endoscope equipped with a raising base (also referred to as a "forceps base") in a parent-and-child type endoscope.

Further, in a case where the endoscope 1 is a cholangioscope as a child endoscope of a parent-and-child type endoscope, it is favorable to cause an angle θ2 formed between an angle of inclination of an inclined face 203 at which an insertion path of the insertion portion 2 is bent in the vicinity of a distal end opening of a channel 102 that is provided in a raising base 201 provided in a distal end portion 200 of a duodenoscope as a parent endoscope that is shown in Fig. 12 and a hole axis X as a central axis of the channel 102 through which the cholangioscope is inserted to substantially match an angle of inclination θ1 (θ1 ≈ θ2) of the inclined portion 24 formed in the distal end portion 6 that is shown in Fig. 11.

By this means, because the inclined portion 24 formed in the distal end portion 6 of the insertion portion of the cholangioscope comes into surface contact with the inclined face 203 of the raising base 201 of the duodenoscope and is guided along the inclined face of the raising base 201, the resistance from the raising base 201 is reduced, and insertion and withdrawal of the insertion portion of the cholangioscope can be smoothly performed, and insertion/withdrawal failures and damage and the like can be prevented.

Note that, the raising base 201 of the duodenoscope may be formed of a material having a small friction coefficient, for example, tetrafluoroethylene (fluorocarbon resin), particularly a material having a low friction coefficient such as PTFE (polytetrafluoroethylene) or PFA (a tetrafluoroethylene perfluoroalkoxyethylene copolymer), and a coating material having a low friction coefficient, for example, chromium plating or a fluorocarbon resin may be coated on the inclined face 203 of the raising base 201.

The invention described in the foregoing embodiment is not limited to the embodiment and modifications described above, and various modifications can be implemented within a range that does not deviate from the spirit of the present invention in the implementing stage. Further, the above described embodiment includes inventions of various stages, and various inventions can be extracted by appropriately combining a plurality of the disclosed configuration requirements.

For example, if the problem to be solved by the invention can be solved and the described effects of the invention are obtained even after omitting some of the configuration requirements from the entire configuration requirements disclosed according to the embodiment, then the configuration obtained by omitting the relevant configuration requirements can be extracted as an invention.

The present application claims priority from Japanese Patent Application No. 2013-110928 filed in Japan on May 27, 2013, the contents of which are hereby incorporated by reference in their entirety into the description of this application, the claims and the drawings.

## Claims

1. An endoscope, comprising:
an insertion portion in which a distal end portion and a bending portion that is bendable in a desired direction are arranged; and
an inclined face that is formed in the distal end portion, the inclined face being formed so as to inclinedly intersect with each side face adjoining a distal end face of the distal end portion, and being formed substantially orthogonal to a ridge line formed intersecting with the distal end face and to a direction in which the bending portion bends, wherein, by contacting against an inner wall of a curved portion of a duct into which the insertion portion is inserted, the inclined face varies a posture thereof so that the insertion portion is rotated and a bending direction of the bending portion matches a curving direction of the curved portion.

2. The endoscope according to claim 1, wherein:
the bending portion bends only in two directions that are point-symmetric directions around a central axis of the insertion portion, and
the ridge line of the inclined face is substantially orthogonal to the two directions.

3. The endoscope according to claim 2, wherein:
among the two directions, the inclined face is formed in a second bending direction that is on an opposite side to a first bending direction in which a curvature at a time of maximum bending of the bending portion is smaller.

4. The endoscope according to claim 2 or 3, further comprising image pickup means for picking up an image of a subject,
wherein the two directions are an upward direction and a downward direction with respect to an observation image of the image pickup means.

5. The endoscope according to claim 1, wherein:
the bending portion bends only in one direction, and
the ridge line of the inclined face is substantially orthogonal to the one direction.

6. The endoscope according to claim 5, further comprising image pickup means for picking up an image of a subject,
wherein the one direction is an upward direction with respect to an observation image of the image pickup means.

7. The endoscope according to any one of claims 1 to 6, wherein the insertion portion is a cholangioscope that is insertable into and withdrawable from inside of a channel of a duodenoscope.

8. The endoscope according to claim 7, wherein an angle formed between an inclined face of a raising base provided in the duodenoscope at which an insertion path of the insertion portion is bent in a vicinity of a distal end opening of the channel and a hole axis of the channel is caused to substantially match an angle of inclination of the inclined face.

9. The endoscope according to any one of claims 1 to 6, wherein the bending portion is passively bendable in the desired direction.
